# EUROPEAN PATENT APPLICATION

(11) **EP 1 814 050 A2**
(43) Date of publication of application: **01.08.2007**
(21) Application number: 06124636.9
(22) Date of filing: 23.11.2006
(51) Int. Cl.: G06F 19/00

(54) **Methods and systems for facilitating planning of surgical procedures**

(30) Priority: 23.11.2005 US 286547
(71) Applicant: General Electric Company, Schenectady, NY 12345 (US)
(72) Inventor: Mahesh, Prakash, Schaumberg, IL 60193 (US); Morita, Mark M., Arlington Heights, IL 60004 (US)
(74) Representative: Illingworth-Law, William Illingworth

(57) **Abstract**

Certain embodiments of the present invention provide a method (200, 600) for performing surgical planning including: providing for display a data set (300, 402, 404) including a representation of a volume of interest of a patient; providing an interactive tool (406) for use in conjunction with the data set (300, 402, 404) ; allowing an interaction with the interactive tool (406) and a portion of the data set (300, 402, 404); and forming a prediction for an effect on a portion of the data set (300, 402, 404) based at least in part on the interaction. In an embodiment, the method (200, 600) further includes selecting the interactive tool (406) from a plurality of tool types. In an embodiment, the variety of tool types includes a plurality of tool tips. In an embodiment, providing an interactive tool (406) for use in conjunction with the data set (300, 402, 404) is performable automatically. In an embodiment, allowing an interaction with the interactive tool (406) and a portion of the data set (300, 402, 404) is performable automatically. In an embodiment, the prediction is storable for later retrieval. In an embodiment, a user is allowed to interact with the interactive tool (406) and the portion of the data set (300, 402, 404).

## Description

### BACKGROUND OF THE INVENTION

Embodiments of the present application relate generally to facilitating planning of surgical procedures. Particularly, certain embodiments relate to providing systems and methods for creating a pre-operative surgical plan for efficiently performing surgery.

Surgery may have associated risks. It may, therefore, be desirable to both clinicians and patients to reduce both the magnitude and probability of any such surgical risks. One way to reduce risk may be to improve pre-operative planning. Improved pre-operative planning may reduce the time for a procedure, and the number of invasive actions which may be performed, for example. Additionally, improved pre-operative planning may decrease any risk of interference with healthy, sensitive tissues and/or organs (e.g. blood vessels or nerves) in the potential path of surgical instruments. Furthermore, certain techniques for improving radiological image quality, such as administration of a contrast agent to a patient, may not be employed during surgery. Therefore, a surgeon or other clinician may employ such techniques during a planning stage to better ascertain the nature of a patient's anatomy.

Pre-operative planning, however may be time consuming. Furthermore, clinicians may lack tools that provide readily obtainable information for pre-operative planning. While a clinician may have access to a radiological image of the patient, the image may require significant analysis by clinicians. Manual analysis of radiological images may be time consuming and expensive. Additionally, manual analysis may not result in electronically available plans capable of real-time interaction, such as during surgery. Furthermore, manual analysis may be difficult, because three dimensional information is generally being shown to the clinician on a display which is only two dimensional.

One particular type of procedure in need of improved pre-operative planning may be called ablation. Ablation may be any surgical excision of tissue, such as a tumor, or a portion thereof. One form of ablation involves insertion of an ablation tool into a tissue to be removed. The ablation tool tip may then achieve a high temperature for a particular duration, thereby causing the tissue to be killed. In thermal ablations, the tissue may be essentially boiled whereas in cryoablation, the tissue may be frozen and killed. Various tool tips may be available, each tip capable of removing a different amount of tissue under various circumstances. It may be difficult for clinicians to calculate the volumetric effects of various ablation tools during pre-operative planning.

Thus, there is a need for methods and systems that reduce risks associated with surgical procedures. Additionally, there is a need for methods and systems that improve the speed and accuracy of pre-operative planning. There is a need for methods and systems that automatically provide pre-operative plans for clinical review.

### BRIEF SUMMARY OF THE INVENTION

Certain embodiments of the present invention provide a method for performing surgical planning including: providing for display a data set including a representation of a volume of interest of a patient; providing an interactive tool for use in conjunction with the data set; allowing an interaction with the interactive tool and a portion of the data set; and forming a prediction for an effect on a portion of the data set based at least in part on the interaction. In an embodiment, the method further includes selecting the interactive tool from a plurality of tool types. In an embodiment, the variety of tool types includes a plurality of tool tips. In an embodiment, the providing an interactive tool for use in conjunction with the data set is performable automatically. In an embodiment, allowing an interaction with the interactive tool and a portion of the data set is performable automatically. In an embodiment, the prediction is storable for later retrieval. In an embodiment, a user is allowed to interact with the interactive tool and the portion of the data set. In an embodiment, a user selects the interactive tool from a plurality of tool types. In an embodiment, the prediction is based on at least one of: a type of the tool, a tip of the tool, a tissue in a portion of the data set, a temperature of the tip of the tool, a duration of the tool in a portion of the data set, a position of the tool in a portion of the data set, and an angle of the tool with respect to a portion of the data set. In an embodiment, the data set includes radiological image data and a segmentation. In an embodiment, the interactive tool is an ablation tool. In an embodiment, a portion of the data set includes data representative of a pathology. In an embodiment, the pathology includes a tumor.

Certain embodiments of the present invention provide a system for performing surgical planning including: a processor; an application executable, at least in part, on the processor, the application capable of receiving a data set representative a volume of interest of a patient; and an interactive tool integratable with the application, wherein a user is capable of performing an interaction through the application and the interactive tool set with the data set to plan a medical procedure involving the volume of interest of the patient. In an embodiment, the data set includes a segmentation. In an embodiment, the segmentation corresponds to at least one of: a pathology, an organ, and a tissue type. In an embodiment, the data set includes at least three-dimensional information. In an embodiment, the interactive tool includes a plurality of tools. In an embodiment, the application is capable of predicting how the interactive tool affects at least a portion of the volume of interest based at least on the interaction to form a prediction. In an embodiment, the prediction is based at least on one of: a type of the tool, a tip of the tool, a tissue in a portion of the volume of interest, a temperature of the tip of the tool, a duration of the tool in a portion of the volume of interest, a position of the tool in a portion of the volume of interest, and an angle of the tool with respect to a portion of the volume of interest. In an embodiment, the interactive tool includes an ablation tool.

Certain embodiments of the present invention provide a computer-readable storage medium including a set of instructions for a computer, the set of instructions including: a provision routine for providing for display a data set including a representation of a volume of interest of a patient; a provision routine for providing an interactive tool for use in conjunction with the data set; an allowance routine for allowing an interaction with the interactive tool and a portion of the data set; and a formation routine for forming a prediction for an effect on a portion of the data set based at least in part on the interaction. In an embodiment, the set of instructions further includes a selection routine for selecting the interactive tool from a plurality of tool types. In an embodiment, the provision routine for providing an interactive tool for use in conjunction with the data set is performable automatically. In an embodiment, the allowance routine for allowing an interaction with the interactive tool and a portion of the data set is performable automatically. In an embodiment, the prediction is storable for later retrieval. In an embodiment, the prediction is based on at least one of: a type of the tool, a tip of the tool, a tissue in a portion of the data set, a temperature of the tip of the tool, a duration of the tool in a portion of the data set, a position of the tool in a portion of the data set, and an angle of the tool with respect to a portion of the data set. In an embodiment, the data set includes radiological image data and a segmentation. In an embodiment, the interactive tool is an ablation tool. In an embodiment, a portion of the data set includes data representative of a pathology.

### BRIEF DESCRIPTION OF SEVERAL VIEWS OF THE DRAWINGS

FIG. 1 shows a system for performing surgical planning, in accordance with an embodiment of the present invention.
FIG. 2 shows a method for performing surgical planning, in accordance with an embodiment of the present invention.
FIG. 3 shows an example of segmentation, in accordance with an embodiment of the present invention.
FIG. 4 shows an example of an application display displaying data and an interactive tool, in accordance with an embodiment of the present application.
FIG. 5 shows an example of prediction forming, in accordance with an embodiment of the present invention.
FIG. 6 shows a method for performing automated surgical planning, in accordance with an embodiment of the present invention.

The foregoing summary, as well as the following detailed description of certain embodiments of the present application, will be better understood when read in conjunction with the appended drawings. For the purpose of illustrating the invention, certain embodiments are shown in the drawings. It should be understood, however, that the present invention is not limited to the arrangements and instrumentality shown in the attached drawings. Further, some figures may be representations of the type of display and/or output associated with methods and systems of the present invention, in accordance with one or more embodiments.

### DETAILED DESCRIPTION OF THE INVENTION

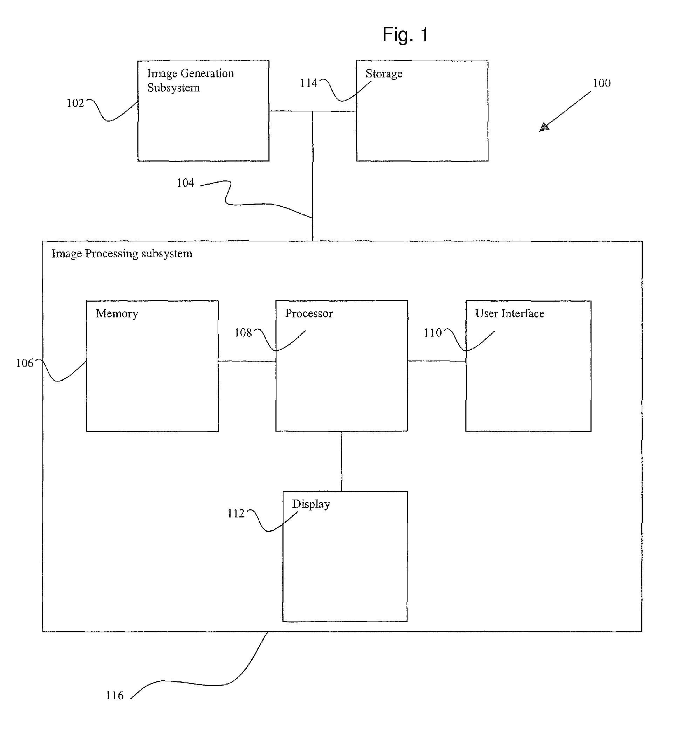

FIG. 1 shows a system 100 for performing surgical planning, in accordance with an embodiment of the present invention. A system 100 may include an image generation subsystem 102 communicatively linked to an image processing subsystem 116 and/or a storage 114 through one or more communications links 104. One or more components, such as storage 114, may be omitted from system 100, for example. One or more components may be integrated in various forms, or may be distributed across a plurality of components in various forms, for example.

An image generation subsystem 102 may be any radiological system capable of generating two-dimensional, three-dimensional, and/or four-dimensional data corresponding to a volume of interest of a patient. A volume of interest of a patient may include tissue, organs, fluids, pathologies (e.g. tumors, abscesses, cysts, etc.), and/or the like. Some types of image processing subsystems 102 include computed tomography (CT), magnetic resonance imaging (MRI), x-ray, positron emission tomography (PET), tomosynthesis, and/or the like, for example. An imaging modality, such as CT, may be enhanced through a contrast agent administered to a patient, for example. An image generation subsystem 102 may generate one or more data sets corresponding to an image which may be communicated over a communications link 104 to a storage 114 and/or an image processing subsystem 116.

A storage 114 may be capable of storing set(s) of data generated by the image generation subsystem 102. The storage 114 may be, for example, a digital storage, such as a PACS storage, an optical medium storage, a magnetic medium storage, a solid-state storage, a long-term storage, a short-term storage, and/or the like. The storage 114 may be integrated with image generation subsystem 102 or image processing subsystem 116, for example. The storage 114 may be locally or remotely located, for example. The storage 114 may be persistent or transient, for example.

An image processing subsystem 116 may further include a memory 106, a processor 108, a user interface, 110 and/or a display 112. The various components of an image processing subsystem 116 may be communicatively linked. Some of the components may be integrated, such as, for example processor 108 and memory 106. An image processing subsystem 116 may receive data corresponding to a volume of interest of a patient. Data may be stored in memory 106, for example.

A memory 106 may be a computer-readable memory, for example, such as a hard disk, floppy disk, CD, CD-ROM, DVD, compact storage, flash memory, random access memory, read-only memory, electrically erasable and programmable read-only memory and/or other memory. The memory 106 may include more than one memory for example. The memory 106 may be able to store data temporarily or permanently, for example. The memory 106 may be capable or storing a set of instructions readable by processor 108, for example. The memory 106 may also be capable of storing data generated by image generation subsystem 102, for example. The memory 106 may also be capable of storing data generated by processor 108, for example.

A processor 108 may be a central processing unit, a microprocessor, a microcontroller, and/or the like. The processor 108 may include more than one processors, for example. The processor 108 may be an integrated component, or may be distributed across various locations, for example. The processor 108 may be capable of executing an application, for example. The processor 108 may be capable of executing methods, such as method 200, in accordance with the present invention, for example. The processor 108 may be capable of receiving input information from a user interface 110, and generating output displayable by a display 112, for example.

A user interface 110 may include any device(s) capable of communicating information from a user to an image processing subsystem 116, for example. The user interface 110 may include a mouse, keyboard, and/or any other device capable of receiving a user directive. For example, the user interface 110 may include voice recognition, motion tracking, and/or eye tracking features, for example. The user interface 110 may be integrated into other components, such as display 112, for example. As an example, the user interface 110 may include a touch responsive display 112, for example. Through user interface 110, a user may be capable of interacting with an application executing on processor 108, for example. Through user interface 110, a user may be capable of interacting with a data set storable in memory 106, for example. Through user interface 110, a user may be capable of interacting with an image displayable on display 112, for example.

A display 112 may be any device capable of communicating visual information to a user. For example, the display 112 may include a cathode ray tube, a liquid crystal diode display, a light emitting diode display, a projector and/or the like. The display 112 may be capable of displaying radiological images and data generated by image processing subsystem 116, for example. The display may be two-dimensional, but may be capable of indicating three-dimensional information through shading, coloring, and/or the like.

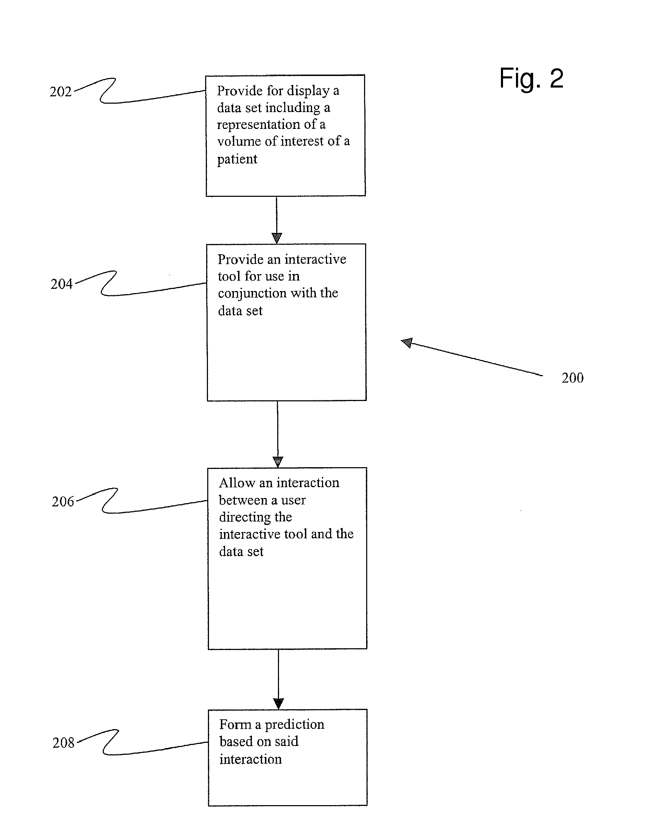

FIG. 2 shows a flowchart of a method 200 for performing surgical planning, in accordance with an embodiment of the present invention. The steps of method 200 may be performed in an alternate order as shown, for example. At least some of the steps of method 200 may be performed simultaneously or substantially simultaneously, for example. Furthermore, some steps of method 200 may be omitted, for example. The steps of method may be performed by a computer and/or other processor (such as processor 108 in FIG. 1) executing a set of instructions on a computer-readable medium, for example. Further, some steps of method 200 may be interchanged with similar steps in method 600, described below, and vice versa.

At step 202, data including a representation of a volume of interest of a patient is provided for display. For example, data may be provided for display on a display (e.g., display 112). For example, data may be generated by radiological imaging (e.g., image generation subsystem 102), and may include information representative of a volume of interest in a patient. Data may contain two, three, and/or four dimensional information, for example. Data may include one or more views of a volume of interest, such as axial, coronal, sagittal, and/or oblique views, for example. Data may be helpful to clinicians for planning and/or visualizing surgical procedures in two, three, and/or four dimensions, for example. For example, data may be helpful to an interventional clinician, such as an interventional radiologist, for planning interventional procedures.

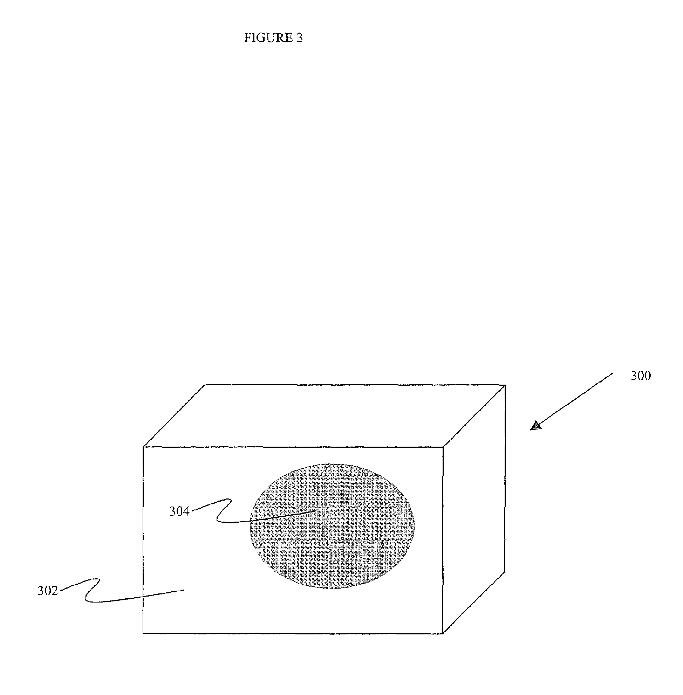

Turning for a moment to FIG. 3, an example of image 300 including segmentation is shown in accordance with an embodiment of the present invention. Data may include data from a radiological imaging system, and additional data, for example. In an embodiment, data includes segmentation 304 of biological structure represented in a radiological image 300. Segmentation 304 may include shapes or forms indicative of various biological structure, for example. For example, segmentation 304 may include an outlined form of a pathology, such as a tumor. Segmentation 304 may include forms indicative of organs and other tissues, such as blood vessels and/or nerves, for example.

Radiological image data 300 may contain a patient's anatomy, including a portion suitable for segmentation 304. Further, a segmentation 304 is shown within the volume of interest 302. The segmentation 304 may be generated by an application running on a processor, such as processor 108 shown in FIG. 1, for example. The segmentation 304 may represent forms of various biological structure, such as organs, tissues, and/or pathologies (e.g. tumors, cysts, etc.), for example. The process of segmentation 304 may be facilitated during image generation by administering an image enhancing agent to a patient, such as a contrast agent, for example.

Segmentation 304 may be formed based on varying intensity properties of pixels and/or voxels, for example. Various tissue, fluid, and/or organ types may be identified based on intensity properties of pixels and/or voxels, for example. A tissue type, such as bone, may cause pixels and/or voxels to have intensity properties within a range associated with bone, for example. A different tissue type, such as nerves, may cause pixels and/or voxels to have intensity properties within a range associated with nerves, for example. Various techniques may be employed to alter intensity properties associated with various anatomy, such as administering a contrast agent to a patient before imaging, for example. A contrast agent may be useful for altering intensity properties such that the intensity properties of various anatomy may be easier to differentiate. In other words, based on associated intensity properties, it may be easier to differentiate various anatomy in an image of a patient with a contrast agent than an image of a patient without a contrast agent, for example.

Based on expected intensity properties associated with pixels and/or voxels, it may be possible to filter various portions of an image. For example, if certain anatomy portions are not to be segmented, such portions may be filtered. For example, musculature, bone, blood vessels, nerves, and/or the like may be filtered, leaving behind a pathology, such as a tumor, for example. Alternatively, anatomy portions may be selected for segmentation based on associated intensity properties, for example. Blood vessels, for example, may be selected for segmentation based on associated intensity properties.

Once selected, a portion of anatomy may be segmented, for example. Various techniques may be used for segmentation, such as edge detection, for example, to form a segmentation 304. Segmentation 304 may be performed in two, three, and/or four dimensions, for example. The segmentation 304 may be two, three, and/or four dimensional, for example. Further processing and interaction may be performed with segmentation 304, for example. The segmentation 304 may be storable in memory (such as memory 106, for example) as a separate data set, or integrated and/or in association with the radiological image data, for example.

Turning back to FIG. 2, at step 204, an interactive tool is provided for use in conjunction with the data set (e.g. radiological image data 302 and segmentation 304). An interactive tool may be one or more tools with which a user may interact. For example, a user through a user interface (such as user interface 110) may select an interactive tool.

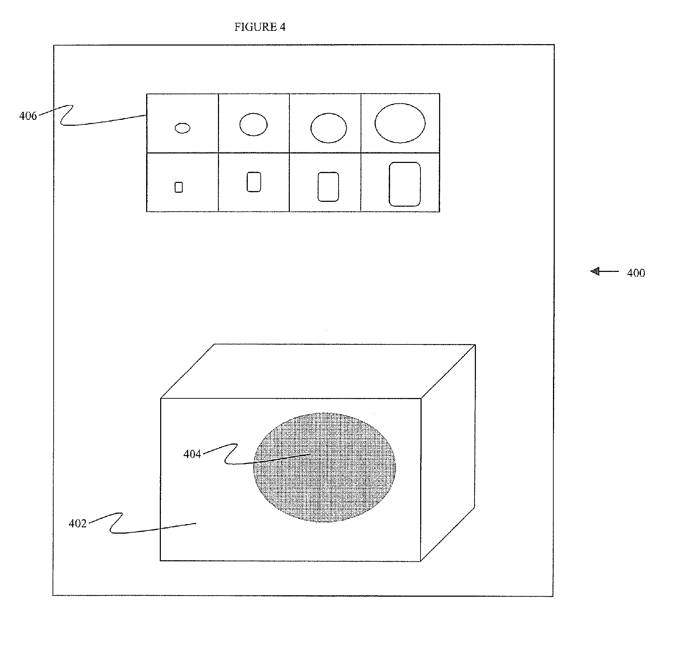

Turning for a moment to FIG. 4, an example of an application display 400 is shown displaying data and an interactive tool 406, in accordance with an embodiment of the present application. Interactive tool 406 selection may be provided through an icon, a menu, a floating menu, a contextual menu, and/or the like, for example. The interactive tool 406 may include a variety of tools, for example. The interactive tool 406 may include one or more tools selectable by a user, for example. The interactive tool 406 may have one or more tool tips for selection, for example. A tool tip may have a variety of sizes, shapes, diameters, and/or the like. A tool tip may impact a three-dimensional volume in a particular manner. The interactive tool 406 may also have other editable parameters, such as tool temperature, tool suction, duration of activation for a tool, and/or the like. In an embodiment, the interactive tool 406 is an ablation tool with a variety of tool tips. Each tool tip may impact surrounding anatomy in a differing way.

Turning back to FIG. 2, at step 206, an interaction is allowed between a user directing the interactive tool (such as tool 406) and the data set (such as the radiological image data 302 or 402 and/or a segmentation 304 or 404). A user may interact with the interactive tool and data set in two, three, and/or four dimensions, for example. A user may be able to position the interactive tool and/or activate the interactive tool, for example. If the interactive tool is an ablation tool, the user may be able to position the tool tip within radiological image data (e.g. 302/402) and/or a segmentation (e.g. 304/404), for example. Once in a satisfactory position, the user may then be able to activate an ablation tool, thereby causing a simulation that heat is provided through the tool tip, for example.

An application may be able to record the interaction of the user, and store in memory the interaction as part of a surgical planning analysis, for example. A user may be able to edit the surgical planning analysis by adding or removing, or otherwise altering interactions, for example. The surgical planning analysis may be storable in memory for subsequent use as a separate data set, or integrated and/or otherwise associated with the underlying radiological image and/or segmentation, for example.

For example, an interaction may be stored as a vector-based trajectory. A trajectory may help a user, such as an interventional radiologist, visualize an efficient path to insert an interactive tool, such as an ablation tool, while avoiding major anatomy. The trajectory may be displayed back to a user, for example, in real-time and/or otherwise.

At step 208, a prediction based on the user interaction is formed. A prediction may be based on the type of interactive tool (e.g. ablation tool), the type of tool tip for the interactive tool, the nature of the interaction, the heat of the interactive tool and/or tool tip, the position of the tool with respect to the region of anatomy, the angle of the tool with respect to the region of anatomy, the duration of tool activity, the type of anatomy in the region of interaction, and/or the like, for example. For example, an ablative tool may burn through certain types of anatomy more quickly and effectively than other types of anatomy. For example, larger tool tips for an ablative tool may burn through larger areas of anatomy. The application may be capable of recognizing some or all of these various factors, and predicting in response how the patient's anatomy will respond to the proposed interaction. Furthermore, the application may be capable of storing the prediction in memory. Further, a prediction may be displayable back to the user. Prediction feedback may be displayed to a user in real-time, for example. An application may record and store a prediction as part of a surgical planning analysis, for example, or as a separate data set, for example. A user may be able to edit the surgical planning analysis by adding or removing, or otherwise altering predictions, for example.

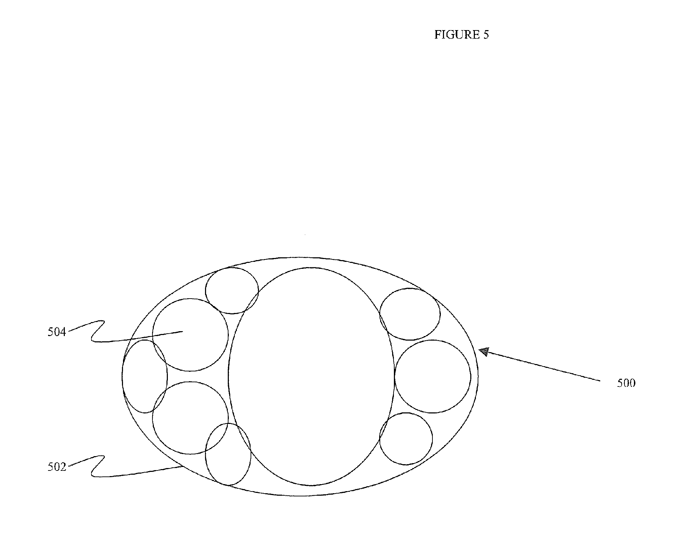

Turning for a moment to FIG. 5, an example of prediction forming 500 is shown, in accordance with an embodiment of the present invention. A segmentation 502 is shown. The segmentation 502 may be a segmentation of a tumor for example. Further, a number of varying predictions 504 based on user interactions are shown. Each prediction 504 may result from a user interaction with an interactive tool, such as an ablation tool. The interactive tool may have a variety of tool tips, for example, thus resulting in the variety of predictions 504, for example. The predictions 504 may be displayed to a user, and further stored as part of surgical planning analysis, for example.

Turning back to FIG. 2, as an illustrative example, method 200 may be performed in the following manner. A patient has a tumor which needs to be removed through ablation (e.g. thermal ablation or cryoablation). At step 202 an application displays data of a patient's anatomy including a tumor, and a corresponding segmentation of the tumor. A CT image of the patient was previously generated in three dimensions after the patient received a contrast agent. The image was transferred to a storage (such as storage 114), and was retrieved by a processor (such as processor 108) executing the application. The application was able to segment the tumor by an imaging protocol which filters non-tumor anatomy portions, based on corresponding intensity properties of the voxels. A shape was then fitted to the tumor tissue using an edge detection algorithm. The segmentation is displayed to the user through the application.

At step 204, the user is provided with an interactive ablation tool with a variety of tool tips through an interactive menu. The tool tips range in size and shape. A user may select one tool tip at a time. A user selects a tool tip through a user interface (such as user interface 110).

At step 206, the user interacts with the radiological image data and the segmentation of the tumor with the ablation tool and selected tool tip. The user interacts with the image and the segmentation through a user interface (such as user interface 110). As the user interacts with the data in the application, the interactions are recorded as part of a surgical planning analysis. As the ablation tool crosses through non-tumor anatomy to reach the tumor, the interaction is recorded. Once the ablation tool tip enters a region of the tumor, the user further interacts with the data by indicating that the ablation tool tip is to be heated. The user may indicate tool tip heating through, for example, clicking on a mousing device, for example.

At step 208, a prediction is formed based on the interaction at step 206. In this particular example, the application is designed to provide as much real-time feedback as possible to the user interacting with the data. Therefore, after every interaction, a prediction is made and displayed back to the user in real-time. Thus, each time the ablation tool crosses through non-tumor tissue while the tip is not hot, a resulting prediction of how the interaction impacts the non-tumor tissue is calculated (based on the size and shape of the tool tip, and the surrounding anatomy) and displayed back to the user in real-time. Each time the ablation tool is heated inside a region of the tumor, a prediction is calculated (based on tool tip size, tool tip shape, tool tip temperature, tool tip heating duration, and type of tumor) and displayed back to the user in real-time. The user may then edit the predictions as they are recorded by, for example, adding, deleting, and/or altering the predictions in accordance with clinical objectives. The set of predictions based on interactions is storable as a surgical planning analysis which may be retrieved at a later point in time, such as during or immediately before surgery, for example.

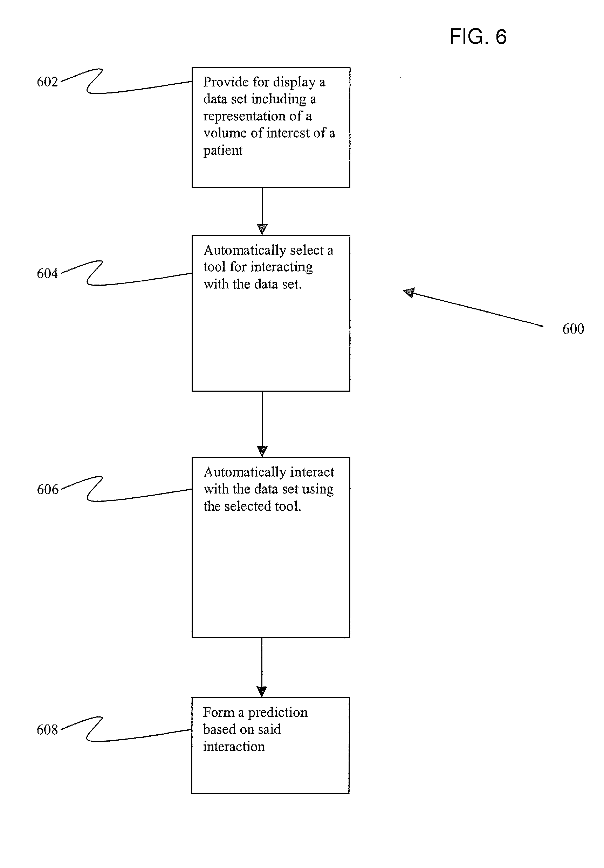

FIG. 6 shows a method 600 for performing automated surgical planning, in accordance with an embodiment of the present invention. The steps of method 600 may be performed in an alternate order as shown, for example. At least some of the steps of method 600 may be performed simultaneously in part, for example. Furthermore, some steps of method 600 may be omitted, for example. The steps of method may be performed by a computer and/or other processor (such as processor 108 in FIG. 1) executing a set of instructions on a computer-readable medium, for example. Further, some steps of method 200 may be interchanged with similar steps in method 600, and vice versa.

At step 602 a data set including a representation of a volume of interest of a patient is provided for display. Step 602 may be similar to step 202, for example.

At step 604, a tool for interacting with the data set is automatically selected. In many respects, step 604 may be similar to step 204. However, a tool may be automatically selected by an application, for example, based on a variety of factors. For example, if a tumor is to be removed through ablation (e.g. thermal ablation or cryoablation), an ablation tool tip size and shape may be selected to minimize the number of ablations needed to remove the tumor substantially. As another example, a tool tip size and shape may be selected automatically based on potential impact to non-tumor tissue along a projected entry path into the tumor tissue. A user may be able to override the automatic selection of an interactive tool, or may otherwise be able to tailor the rules used for automatic selection of the interactive tool, for example.

At step 606 the selected tool automatically interacts with the data set. In many respects, step 606 may be similar to step 206. However, a tool may automatically interact with data through an application, for example, based on a variety of factors. For example, if a tumor is to be removed through ablation (e.g. thermal ablation or cryoablatino), an ablation tool may be guided through non-tissue anatomy along an efficient path into a particular region of the tumor. Once in position, the tool may be automatically actuated for a duration automatically calculated based on efficiency. A user may be able to override the automatic selection of an interactive tool, or may otherwise be able to tailor rules used for automatic selection of the interactive tool, for example. For example, a user may be able to constrain certain parameters such as tool tip size and/or tool tip temperature, while letting automated algorithm(s) determine other factors. Interactions may be stored in memory and/or saved as part of a surgical planning analysis. Interactions may be further edited by a user and/or an automatic planning algorithm by adding, removing, and/or altering interactions, for example.

At step 608 a prediction is formed based on the interaction. Step 608 may be similar to step 208, for example. Method 600 may automatically loop back to step 604 and/or 606 and continue until a particular automated planning and prediction process is complete, for example. Predictions may be stored in memory and/or saved as part of a surgical planning analysis. Predictions may be further edited by a user and/or an automatic planning algorithm by adding, removing, and/or altering predictions, for example.

As an illustrative example, method 600 may be performed in the following manner. A patient has a tumor that needs to be removed through ablation (e.g. thermal ablation or cryoablation). At step 602 an application displays data of a patient's anatomy including a tumor, and a corresponding segmentation of the tumor. A CT image of the patient was previously generated in three dimensions after the patient received a contrast agent. The image was transferred to a storage (such as storage 114), and was retrieved by a processor (such as processor 108) executing the application. The application was able to segment the tumor by an imaging protocol which filters non-tumor anatomy portions, based on corresponding intensity properties of the voxels. A shape was then fitted to the tumor tissue using an edge detection algorithm. The segmentation is displayed to the user through the application.

At step 604, the system automatically chooses an ablation tool tip that may efficiently remove the tumor, based on the perceived size of the tumor (e.g. the segmentation). The user is asked to confirm the choice of tool tips, and the user confirms the automatic selection of the interactive tool. For this example, the same size tool tip and shape will be used for all ablation tool interactions.

At step 606, the application automatically interacts with the radiological image data and the segmentation of the tumor with the ablation tool and selected tool tip. As the application interacts with the data, the interactions are recorded as part of a surgical planning analysis. As the ablation tool crosses through non-tumor anatomy to reach the tumor, the automatic interaction is recorded. Once the ablation tool tip enters a region of the tumor, the application further automatically interacts with the data by indicating that the ablation tool tip is to be heated for a specific duration and temperature.

At step 608, a prediction is formed based on the automatic interaction performed at step 606. For example, each time a tool moves through the image data, and each time the ablation tool is heated inside a region of the tumor, a prediction is calculated (based on tool tip size, tool tip shape, tool tip temperature, tool tip heating duration, and type of tumor). In this example, the display is not updated until the tumor has been substantially ablated, virtually. Therefore, the method 600 loops back to step 606 to perform further iterations until the tumor volume (e.g. segmentation) has been ablated. After all iterations have been performed, the display is updated to indicate all of the predictions that have been calculated and recorded. The user may then edit the predictions after they are recorded by, for example, adding, deleting, and/or altering the predictions in accordance with clinical objectives. For example, the user may perform subsequent iterations of method 200, for example. The set of predictions based on interactions is storable as a surgical planning analysis that may be retrieved at a later point in time, such as during or immediately before surgery, for example.

In an embodiment, an image processing subsystem 116 includes a computer-readable medium, such as a hard disk, floppy disk, CD, CD-ROM, DVD, compact storage, flash memory and/or other memory (such as memory 106). The medium may be in memory 106, processor 108, storage 114, and/or in a separate system. The medium may include a set of instructions capable of execution by a computer or other processor. The providing, display, interacting, selecting, automating, and predicting functions described above may be implemented as instructions on the computer-readable medium. For example, the set of instructions may include a provision routine that provides for display a data set including a representation of a volume of interest of a patient. Additionally, the set of instructions may include a provision routine that provides an interactive tool for use in conjunction with a data set. Additionally, the set of instructions may include an allowance routine that allows an interaction with the interactive tool and a portion of the data set. In an embodiment, an interaction is allowed with a user. In another embodiment, an interaction is allowed to proceed automatically. Additionally, the set of instructions may include a formation routine that forms a prediction for an effect on a portion of the data set based at least in part on the interaction. In an embodiment, the set of instructions may include a selection routine for selecting the interactive tool from a plurality of tool types. In an embodiment, the tool may be selected automatically.

Thus, embodiments of the present application provide methods and systems that reduce risks associated with surgical procedures. Additionally, embodiments of the present application provide for methods and systems that improve the speed and accuracy of pre-operative planning. Moreover, embodiments of the present application provide methods and systems that automatically provide pre-operative plans for clinical review.

While the invention has been described with reference to certain embodiments, it will be understood by those skilled in the art that various changes may be made and equivalents may be substituted without departing from the scope of the invention. In addition, many modifications may be made to adapt a particular situation or material to the teachings of the invention without departing from its scope. For example, features may be implemented with software, hardware, or a mix thereof. Therefore, it is intended that the invention not be limited to the particular embodiment disclosed, but that the invention will include all embodiments falling within the scope of the appended claims.

## Claims

1. A method (200, 600) for performing surgical planning comprising:
providing for display a data set (300, 402, 404) including a representation of a volume of interest of a patient;
providing an interactive tool (406) for use in conjunction with said data set (300, 402, 404);
allowing an interaction with said interactive tool (406) and a portion of said data set (300, 402, 404); and
forming a prediction for an effect on a portion of said data set (300, 402, 404) based at least in part on said interaction.

2. The method (200, 600) of claim 1 further comprising selecting said interactive tool (406) from a plurality of tool types.

3. The method (200, 600) of claim 1, wherein said providing an interactive tool (406) for use in conjunction with said data set is performable automatically.

4. The method (200, 600) of claim 1, wherein allowing an interaction with said interactive tool (406) and a portion of said data set (300, 402, 404) is performable automatically.

5. The method (200, 600) of claim 1, wherein a user is allowed to interact with said interactive tool (406) and said portion of said data set (300, 402, 404).

6. The method (200, 600) of claim 1, wherein said prediction is based on at least one of: a type of said tool (406), a tip of said tool (406), a tissue in a portion of said data set (300, 402, 404), a temperature of said tip of said tool (406), a duration of said tool (406) in a portion of said data set (300, 402, 404), a position of said tool (406) in a portion of said data set (300, 402, 404), and an angle of said tool (406) with respect to a portion of said data set (300, 402, 404).

7. The method (200, 600) of claim 1, wherein said data set (300, 402, 404) includes radiological image data (400) and a segmentation (502).

8. The method (200, 600) of claim 1, wherein said interactive tool (406) is an ablation tool.

9. A system (100) for performing surgical planning comprising:
a processor (108);
an application executable, at least in part, on said processor (108), said application capable of receiving a data set (300, 402, 404) representative a volume of interest of a patient; and
an interactive tool (406) integratable with said application,
wherein a user is capable of performing an interaction through said application and said interactive tool (406) with said data set (300, 402, 404) to plan a medical procedure involving said volume of interest of said patient.

10. The system (100) of claim 9, wherein said application is capable of predicting how said interactive tool (406) affects at least a portion of said volume of interest based at least on said interaction to form a prediction.
